# EUROPEAN PATENT SPECIFICATION

(11) **EP 3 036 321 B1**
(45) Date of publication and mention of the grant of the patent: **09.10.2019**
(21) Application number: 14771498.4
(22) Date of filing: 19.08.2014
(51) Int. Cl.: C12N 5/00

(54) **PROCESS FOR PRODUCING MULTIPOTENT STEM CELLS AND PROGENITORS**
VERFAHREN ZUR HERSTELLUNG MULTIPOTENTER STAMMZELLEN UND VORLÄUFER
PROCESSUS DE PRODUCTION DE CELLULES SOUCHES MULTIPOTENTES ET DE PROGÉNITEURS

(30) Priority: 20.08.2013 BR 102013021202
(43) Date of publication of application: 29.06.2016
(73) Proprietor: CCB - Centro De Criogenia Brasil Ltda., 01430-000 São Paulo - SP (BR)
(72) Inventor: AYOUB, Carlos Alexandre, 04116-000 São Paulo - SP (BR); KERKIS, Alexandre, 05586-060 São Paulo - SP (BR); LIZIER, Nelson Foresto, 18530-000 Tietê - SP (BR)
(74) Representative: KATZAROV S.A.
(86) International application number: PCT/BR2014/000301
(87) International publication number: WO 2015/024089

(56) References cited:
- WO-A1-2005/001081
- WO-A1-2012/149574
- LIZIER N F ET AL: "Scaling-Up of Dental Pulp Stem Cells Isolated from Multiple Niches", PLOS ONE, vol. 7, no. 6, 29 June 2012 (2012-06-29), page e39885, XP55159827, DOI: 10.1371/journal.pone.0039885
- LA ROCCA G ET AL: "Isolation and characterization of Oct-4+/HLA-G+ mesenchymal stem cells from human umbilical cord matrix: differentiation potential and detection of new markers.", HISTOCHEMISTRY AND CELL BIOLOGY, vol. 131, no. 2, February 2009 (2009-02), pages 267-282, ISSN: 1432-119X

## Description

The present invention generally relates to a non-enzymatic process for producing multipotent stem cells and progenitors from the growth of stem cell niches.

### Background of the invention

It is known that stem cell populations are at particular anatomical locations of the human body - i.e., natural niches - which guarantee their maintenance and the cell interactions necessary to allow division of these cells and participation in appropriate homeostasis and tissue repair. Thus, the stem cell niche is the functional unit of any tissue containing several types of cells residing in harmonic interaction with its extracellular matrix, in a microenvironment that provides short-range intercellular signals for maintenance of the non-differentiated state of cells.

In this invention, in a particular and non-exclusive manner, suitable stem cell niches comprise those typically included in extraembryonic tissues expelled by a woman body at childbirth, particularly the placenta, amniotic membrane and the umbilical cord, including its constituents (for example, veins, arteries, epithelium and connective tissue), preferably after blood removal.

In the text that follows, just for ease of expression and as representative of natural stem cell niches (NSCN) adequate to the invention, specific references will be made to the intact umbilical cord tissue (UCT), including mesenchymal connective tissue (Wharton's jelly), arteries, vein and outer epithelium, after blood withdrawal, said mention not limiting, in any way, the invention with regard to the use of other NSCN such as, for example, nerve, muscle, fat, bone, skin and organ-derived (e.g., liver, lungs, heart, spleen, liver, pancreas, testes, ovaries or even biopsy-derived) tissues, but without being limited by these included, as well as other postnatal and adult tissues, or even cancer tissue with NSCN.

Various isolation methods of stem cell from human tissues or substrates are known. However, until now, no method can guarantee the obtainment of cells in large-scale in response to the growing demand of stem cells and their derivatives for therapies, and for large-scale production of bioactive molecules (peptides, growth factors, hormones, etc.) that have trophic properties of pericytes and which could replace SC (stem cell) in many therapies.

There are countless mentions in the state of the art to procedures in which various substrates in the human body are submitted to enzymatic processes to obtain and/or retrieve stem cells. However, even with the use of enzymatic processes, there is no disclosure of methods that produce stem cells in large amounts.

It is known that substantial amounts of stem cells are required, among others, for the production of tissues, organ transplants, tissue and/or complete organ printing, healing of an injury directly in the patient and immunosuppression in many autoimmune diseases. There are references in bibliography that these treatments require an amount of 2 x 10⁶ cells per kilogram of patient weight, these stem cells being standardized in relation to ST properties and the number of cell passages.

As will be seen below, the present invention provides a simple process with high yield for obtaining large amounts of stem cells and progenitors, in an essentially non-enzymatic manner.

### Description of the Figure

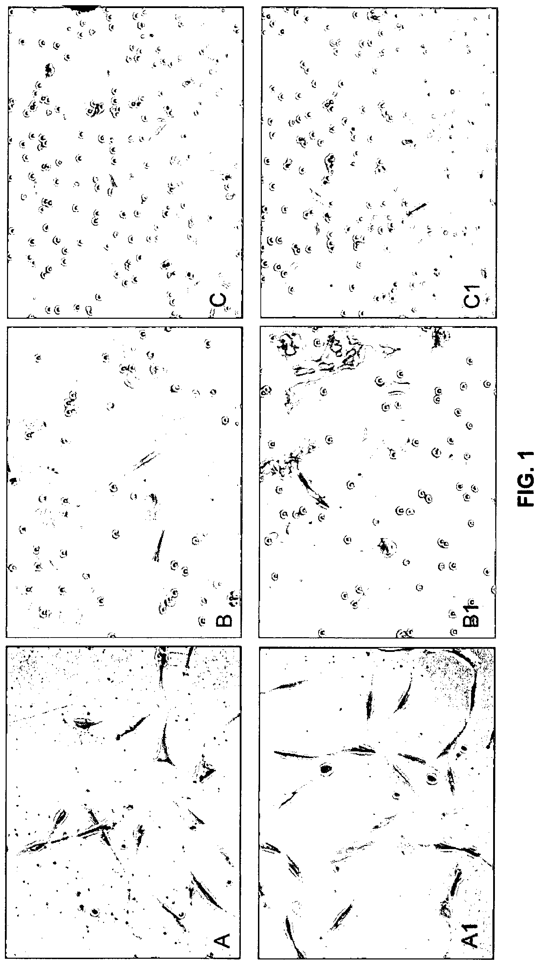

Figure 1 - Cell populations obtained by the inventive process and by 2 alternative enzymatic methods, after 72 hours in cultivation in A, B, C and after 5 days in A1, B1 and C1.

### Description of the Invention

The present invention relates to an innovative process for producing adult stem cells, particularly multipotent mesenchymal/stromal stem cells and perivascular precursors, such as pericytes.

Differently from the prior art processes, the process of the invention is essentially non-enzymatic and is based on the propagation and isolation of population of stem cells that actively proliferate in their niches, in a biologically synergistic manner (that is, in equilibrium with their environment). The way of verifying if a particular tissue is a stem cell niche is not part of the invention, which is known by the person skilled in the art, for example, by immunofluorescence and immunohistochemistry.

The present invention provides an advantageous and robust process for producing cells with 100% of success and effectiveness with respect to the processed tissues, allowing the obtainment of large amounts of cells and minimum time delays related to the isolation process.

The mesenchymal stem cells (MSC) isolated in large amounts from the process of the invention exhibit all the characteristics of mesenchymal stem cells and of pericyte type, either for production of medicinal molecules used directly in therapy or in the pharmaceutical industry for drug production. The SC obtained by the process of the invention presents a large capacity of differentiation (induced or spontaneous) *in vitro* and *in vivo;* with large capacity of tissue regeneration using culture media and agents and/or substrates ("scaffold") in liquid, rigid or gelatinous support phase, or extracellular matrix (complex of macromolecules: fibrous components, proteins and polysaccharides) of diverse nature, natural or synthesized.

A characteristic of stem cells/progenitors produced by the process of the invention is having the same pattern of expression of markers related to the non-differentiated state of *in vivo* cells present within cultivated fragments.

The process of the invention comprises, in an innovative manner, the *in vitro* cultivation of SC niches (NSCN) . Without being a justification of the invention, it is known that the *in vitro* cultivation of stem cells outside their usual niche may induce changes in karyotype or phenotype stability, in the molecular signature and genomic stability. Additionally, due to the unequal division of stem cells (which produce a stem cell and a progenitor), they lose multipotentiality with cell passages (ability to produce a broad spectrum of differentiated cell types). This mixed population of mesenchymal stem cells derived from the natural niche can provide greater biological synergy of cells isolated according to the process of the invention, which dispenses prior removal of blood vessels or any other tissue, only withdrawing the blood present.

A feature of the process of the invention is the floating culture (or 3D) of tissue fragments of NSCN, promoting substantial amounts of stem cells and progenitors substantially free from genetic and biological changes.

According to the invention, the tissue fragments keep floating in basal culture medium, producing stem cells/progenitors stem cells. The release of SC occurs through natural migration of cells from NSCN fragments to the plastic of culture bottle, ensuring that these cells will maintain the homogeneity of molecular profile of the population and survival unchanged after this natural insulation.

It is observed that the culture of floating tissue fragments, according to the invention, typically exhibits the following dynamics in SC production: with the culture of NSCN fragments in suspension, efficient diffusion of gases and nutrients throughout the fragment occurs, the SC leave the state of quiescence and have their proliferation in the niches stimulated (i.e. have their number increased by symmetric division), and their migration also occurs from inside the NSCN fragments to the surface thereof in response to tissue fragmentation. Hence, the fragments become denser and temporarily touch/adhere to the culture bottle substrate, releasing the SC to migrate out of the debris and adhere to the substrate. After adhesion of debris to the substrate, an increase of migration to the detriment of proliferation occurs, since the diffusion of nutrients/gases apparently becomes less intense. The fragment then tends to become lighter due to the extensive migration of SC to the substrate and, autonomously or by simple stirring of the culture bottle, the fragment detaches from the substrate and floats. Once floating again, the diffusion of gases and nutrients throughout the tissue is reestablished and a new increase in proliferation of SC in the niches occurs, and the fragment becomes denser again, establishing a new cycle of adhesion and release of SC and subsequent flotation. This flotation-proliferation-sedimentation-adhesion-migration-re lease system can be repeated several times during cultivation of tissue fragments from SC natural niches, producing colonies of stem cells. As time goes by, multiple colonies consisting of inventive cells can be observed at the bottom of the culture bottle.

According to a preferred embodiment, the cell culture at the bottom of the culture bottle - a relatively homogeneous culture of stem cells with stem cell/progenitor characteristics - is kept semi-confluent, particularly with 70% to 90% of confluence, in order to prevent spontaneous cell differentiation. The term "semi-confluent" means a culture that is not so dense in a manner that would allow substantial contact of cells with each other, as is verified in high density confluent cultures.

A particular embodiment of the invention additionally includes modifications of the basal culture medium, by the addition of growth factors and/or other bioactive molecules that may alter the characteristics of cells arising from the process.

In a particular embodiment of the invention, the NSCN tissue to be cultivated may be submitted to an initial mild pretreatment with dissociation enzymes (for example, trypsin, collagenase, TrypLE™, etc.) before the immersion of these tissues in culture medium. Such pretreatment does not aim at an extensive digestion of the tissue, but only at a small relaxation of tissue coherence to facilitate the movement of SC when immersed in the basal culture medium.

After the end of the invention process, further enzymatic treatment of adhered SC and their respective cell passages to other vessels for *in vitro* or ex *vivo* growth or expansion of the resulting stem cells/progenitors favors isolation of pericytes.

A peculiarity of the process of the invention is the removal and transfer of fragments from NSCN, for example, by any adequate mechanic manner, for example, with the aid of devices such as a pipette, clamp, needle, or appliances, or simply by pouring the contents from a container to another. After transferring these NSCN tissue pieces, (floating fragments) to a new container, they can continue the production of SC with passage zero (without passage), since no enzymatic treatment was performed.

The process of the invention uses NSCN fragments, which comprise stem cells that proliferate within the tissue and keep expressing markers of various types of stem cells even after several mechanical tissue transfers mentioned.

The process of the invention is minimally invasive on the use of niches comprised in extraembryonic tissues expelled by a woman body at childbirth, particularly the umbilical cord, amniotic membrane and placenta. Among other advantages of stem cells contained, their youth should be mentioned. Mammalian aging is associated with a reduction in tissue regeneration, increase of occurrence of degenerative diseases and cancer. Since stem cells regenerate many adult tissues and when these, by accumulation of mutations, can contribute to cancer development, age-related modifications in stem cells probably contribute to age-related morbidity. Consistent with this, the role of stem cells in various tissues decreases with age, possibly resulting in the loss of expression of tumor suppressors, DNA damage, changes in cell physiology and changes in the tissue environment. It remains unknown whether declines in stem cell function during aging influence the organism longevity; however, the mechanisms that influence longevity also modulate age-related morbidity, in part, through effects on stem cells. Therefore, the stem cells of extraembryonic tissues related to childbirth have extreme importance to cell therapy, since they are young cells - highlighting the fact that cells obtained by the process of the invention are not embryonic cells.

Although it is not essential, an initial wash of NSCN tissue is appropriate to the process of the invention, particularly for blood removal before the fragmentation of tissue that will grow.

Particularly, NSCN wash is made externally and internally. It is externally made, for example, with distilled water or sterile buffered saline solution (PBS or physiological solution and antibiotics (such as penicillin and streptomycin 2%)). It is internally made, for example, by washing the inside of the blood vessels (arteries and veins) with distilled or sterile buffered saline solution (PBS or physiological solution and antibiotics (such as penicillin and streptomycin 2%).

The fragmentation of NSCN to be cultured according to the process of the invention can be made from any suitable way, for example, with sharp blades, by manual or mechanized cutting, under sterile conditions. Preferably, but not essentially, the cut must be made with little pressure on the tissue, without heating, adequately with a water jet cutter under pressure.

In a particular embodiment, the present invention also relates to repetitive cryopreservation/thawing of tissue fragments of NSCN(s), thus allowing the continued production of new stem cells/progenitors in high-scale, particularly when involving NSCN tissue from a single patient and in low cell passages (typically less than or equal to 5). However, this aspect does not limit the process of the invention, which can use NSCN from a single patient, or from two or more distinct patients, or can concomitantly use different NSCNs from one or more patients, or with more NSCN passages.

The cells obtained in accordance with the process of the present invention express, in the non-differentiated state, a group of MSC markers (CD29, CD73, CD90, CD105) concurrently with pericyte markers (CD140b and CD166) and neither express hematopoietic lineage markers (CD34 and CD45) nor histocompatibility markers (HLA-DR). The cells obtained from the process of the invention are multipotent, capable of self-renewal with continuous proliferation and *in vitro* differentiation, with the use of known conditions, for example, addition of inducing agents (for example, retinoic acid, dimethyl sulfoxide, etc.),growth factors and cytokines. Under these conditions, the stem cells/progenitors of the invention differentiate into distinct cell types, such as bone, cartilage and fat. Since stem cells/progenitors obtained according to the invention also express CD31 marker, which is characteristic of endothelial progenitor cells, they can additionally differentiate into muscle, endothelial and nerve cells.

Cells and/or fragments obtained by the process of the invention are suitable in many possibilities of use, such as in therapeutic, non-therapeutic, biotechnological and pharmaceutical uses.

Thus, the present invention aims at a process for obtaining stem cells, particularly multipotent stem cells/progenitors from the culture of NSCN tissue, characterized by comprising the following steps:
A - obtainment of one or more NSCN;
B - pre-preparation of one or more NSCN;
C - preparation of tissue fragments of one or more NSCN;
D - promotion of SC propagation by culturing NSCN fragments in a basal culture medium;
E - separation of SC from NSCN fragments;
F - optionally, separated NSCN fragments in E return to step D.

The mention of SC in process steps includes stem cells and progenitors.

Step A to the process of the invention is particularly performed with the obtainment of a particular NSCN, such as umbilical cord. Accordingly, without excluding any other, the NSCN(s) used in the process of the invention are tissues from woman childbirth, particularly umbilical cord, amniotic membrane and placenta, even more particularly the intact umbilical cord, with arteries and vein, Wharton's jelly and epithelium. Nervous, muscle, fat, bone, skin and organ-derived tissues, such as of liver, lungs, heart, spleen, liver, pancreas, testes, ovaries or even biopsy-derived tissues, are adequate to the invention, but are not limited by these included, as well as other postnatal and adult tissues, and even cancerous tissue having NSCN.

According to item (B) above, NSCN is submitted to a pre-preparation, which may consist of cleaning, washing, tissue pre-cuts, grinding, compression, mild pretreatment with dissociation enzymes (collagenase, dispase, trypsin, TrypLE™, among others), etc.

Particularly, the NSCN tissue is submitted to cleaning/washing, aiming at blood removal and other substrates that may adversely affect SC propagation during cultivation, for example, by induction of differentiation, intoxication, contamination, etc. In the particular case of umbilical cord, proper cleaning is carried out both internally and externally, for example, with one or more of distilled water, saline solution, physiological solution and antibiotics (for example, penicillin and streptomycin) . Internal washing is carried out, for example, by injecting the substrate into the inner tissue vessels, and removing the material thus dragged.

In a particular embodiment, the washing and cleaning of umbilical cord is carried out, for example, in pre-cut parts of 5 to 10 cm from the umbilical cord vein, avoiding the inclusion of blood clots.

Still in accordance with step B, the pre-preparation may involve a mild initial pretreatment of the tissue with dissociation enzymes (for example, trypsin, collagenase, TrypLE™, etc.). Such pretreatment does not aim at the extensive digestion of the tissue, but only at a small relaxation of tissue coherence to facilitate the movement of SC when immersed in the basal culture medium.

In relation to step C of the process of the invention, a manner to fragment the NSCN tissue, as already mentioned, is in any way suitable for its purpose. In the case of umbilical cord, fragments obtained from transversal and/or longitudinal cuttings until reach, for example, cubes with dimensions from 0.5 to 1 cm, are adequate. Any other size or fragment format is included in the scope of the invention, even particles obtained by tissue grinding.

Still in relation to step C, in a particular embodiment of the invention, the NSCN fragmentation aims at using only specific parts of intact tissues for growth in the following step D. For example, in the case of umbilical cord, fragments exclusively of the veins, arteries, epithelium or Wharton's jelly may be used, each one generating particular stem cells/progenitors. They are fragments from NSCN constituents (vein, artery, epithelium, etc.) that can be used separately, according to the desired purpose, for producing cells with molecular signature, differentiation potential and production of specific active molecules, or can be used together.

In relation to step D of the process of the invention, the basal medium for culturing fragments of umbilical cord and/or NSCN cells is anyone that allows the propagation/expansion and isolation of stem cells and progenitors with the same phenotypic and molecular characteristics. For example, an adequate medium is DMEM/F12 ("Dulbecco's modified Eagle's medium"/Ham's F12, 1:1, of Invitrogen, USA) or any equivalent substrate known by a person skilled in the art, typically containing amino acids, proteins, serum and antibiotics.

Said basal culture medium adequately comprises, for example, ± 15 wt% serum. Particularly, said serum is bovine derived such as, for example, bovine fetal serum, and human sera are also adequate (for example, platelet-rich or -poor plasma) and other animals, including mixtures thereof, as well as other natural or synthetic reagents which may allow SC isolation.

The culture medium of step D adequately contains antibiotic and/or amino acids. The antibiotics used are comprised in the technical knowledge of the skilled technician, for example, a combination of penicillin and streptomycin or gentamicin. Amino acids useful for carrying out the invention are glutamine, non-essential amino acids or mixtures thereof, among others.

The frequent exchange/renewal of culture medium is particularly carried out in step D, since the fragments of NSCN tissues spend medium more quickly than cells. The medium exchange as pH changes from basic to acid is appropriate, said change being assessed, for example, by change of the growth medium color, typically from pink to yellow, or in any other appropriate manner.

Optionally, after step D of the process of the invention, a concentrate can be prepared containing NSCN fragments and stem cells obtained (for example, by removing part or all the basal culture medium), such concentrate being preserved for use in a future moment. The preservation of such concentrate is, for example, via cryopreservation; in ways known by a person skilled in the art. The use of this concentrate, in a future moment is properly performed with thawing, new insertion in basal culture medium and subsequent separation of cells/progenitors from fragments, according to item E above.

In a particular embodiment, the concentrate of NSCN and/or stem cells obtained and/or conditioned by culture can also be freeze-dried, with additional uses beyond reuse in the process of the invention (step D).

According to step E of the process of the invention, after one culture cycle, fragments of NSCN tissue are mechanically isolated and can - according to step F- be resubmitted to cultivation of step (D), either immediately subsequent or after storage (cryopreservation). The reuse of fragments in new cultures can be made until exhaustion of the ability to release SC/progenitors. Such fragments from step E can be mixed with new fragments, originated after steps A, B and C, which have not been previously used, for cultivation in step D.

In a particular embodiment, NSCN fragments may also be decellularized, digested and/or freeze-dried after step E, aiming at additional uses besides of reuse, according to step F of the process of the invention.

The treatment that can be given to adhering SC/progenitors after separation of fragments, according to item E above, is known itself by a person skilled in the art. Cells are typically washed, for example, with sterile buffered saline solution, with or without antibiotics, to then carry out their dissociation (since cells are half-confluent), whether by mechanical or enzymatic means, to perform their harvest. Preferably, this dissociation is performed by enzymatic means, particularly by using Tryple™ (marketed by Invitrogen, a US company), which is an exceptionally pure recombinant enzyme free of animal components and mild to cells. A solution of about 0.25-0.05% of trypsin/ethylenediaminetetraacetic acid (EDTA) can be also used, which is marketed, for example, by Sigma-Aldrich. Thereafter, the harvested cells are typically submitted to cell passage by enzymatic means, or cryopreserved for later use, according to processes known by a person skilled in the art.

In a particular embodiment, the SC/progenitors obtained by the process of the invention, isolated according to step E of the process of the invention, may be also submitted to freeze-drying for specific uses. The culture medium conditioned by cells and/or fragments can be also freeze-dried for producing substrates of cultures, bioactive molecules, nutritional supplements and for aesthetic use.

The cryopreservation of tissue fragments of NSCN or SC/progenitors, or mixtures thereof, possible after step E of the process of the invention, is adequately conducted in a freezer, at temperatures around -80 °C and subsequently in liquid nitrogen, at temperatures around -196 °C, according to the knowledge of a person skilled in the art.

After item E of the process of the invention, isolated SC/progenitors are typically submitted to enzymatic cell passage, according to process known by a person skilled in the art, in number adequate to the intended purpose. Great stability of SC markers of the present invention is noted after a large number of passages, for example, 25 or more.

The culturing of NSCN tissue fragments and multiplication by cell passage of SC/progenitors resulting from the process of the present invention can be carried out on microcarriers or in known bioreactors intended for producing cells in high-scale, as is known in the technical field.

### EXAMPLES

Exemplary embodiments of the instant invention are given below in order to illustrate their realization, without imparting any limitations beyond those expressed in the attached claims.

### Example 1

### Processing of umbilical cord and cell culture

The description of this example represents the mean number of countless embodiments performed in the same manner.

From an intact umbilical cord a 5-cm fragment was prepared, which was washed twice inside and out (in this case with a needle on a syringe) with sterile buffered saline solution [0.01 M PBS, pH 7.4] containing antibiotics [100 units/mL penicillin and 100 µg/mL streptomycin] to eliminate, to the maximum possible extent, contamination with blood. In the first internal washings the, washing solution was still contaminated with blood, and had reddish color. Additionally, sterile water injected in the umbilical cord vessels was used, and the washing solution remained colorless. Then, using a scalpel, the cord was cut longitudinally and transversally to obtain about 35 pieces of 0.5 x 0.5 cm approximately, and then they were transferred to a 75 cm² flask (Corning, NY) containing DMEM/F12 medium (Dulbecco's modified Eagle medium/Ham's F12, 1:1, marketed by Invitrogen, a US Company) supplemented with 15% fetal bovine serum (FBS, marketed by Hyclone, a US Company), 100 µg/mL penicillin, 100 µg/mL streptomycin, 2 mM L-glutamine and 2 mM of non-essential amino acids. The bottle was kept in a CO₂ greenhouse under wet atmosphere and at 37 °C. The fragments began to release the cells from days 2-3 or 5-7, an individual variation observed in other embodiments of the process of the invention. Cell culture growth of fragments of umbilical cord was kept in these conditions for two weeks. The medium color remained rose (alkaline) and not yellow (acidic) by daily replacement, and it was verified that the tissue consumes the medium very quickly. The fragments kept floating or wafting in the culture medium. Once the bottle bottom was covered by cells, forming individual half-confluent colonies, in 5-7 or 9-11 days, according to variation observed with different fragments, the fragments were transferred, by simply pouring to another bottle of the same size.

Cells adhered to the bottles had a morphology similar to fibroblasts, high rate of proliferation and nearly 4 x 10⁶ cells were generated from the 35 fragments in 2 weeks, Umbilical cord cells exhibited high capacity of forming individual colonies and, in passage 1, the frequency of formation of cell colonies was around 100 colonies/100 plated cells in a 90 cm² plate. The growth kinetics of a single colony derived from umbilical cord cells was measured at passage 1. During 16 days, cells were collected and counted daily and changes in the growth rate were not observed. Changes in the morphology or growth pattern of these stem cells/progenitors were not verified after 25 tickets either.

These cells showed normal karyotype in all lines obtained and no changes could be observed after 10 passages.

### Example 2 - Comparison of the method of the invention with enzymatic method of state of the art

An umbilical cord was isolated and split into three equal parts (nearly 5 cm). The first part was processed according to Example 1 and was transferred directly to the growth medium. The second and third parts were washed and processed fragmented, as described, in example 1, to then be treated with collagenase (0.1% collagenase for two hours) and TrypLE™ (for 30 minutes), respectively. It can be observed, in figure 1, the difference between cell populations obtained by these three methods, after 72 hours in cultivation in A, B, C and after 5 days in Al, B1 and C1. The difference in cell morphology can be observed, which is more defined and fusiform in A and Al, as well as on the amount of adhered cells that begin to form colonies. The proliferation of these cells was evaluated by plating equal number of cells (10³ on 25 cm²), it being observed that while the inventive cells reached 90% of confluence with an amount of 10⁶ cells in 5 days and were frozen, the cells obtained by enzymatic methods reached only 70% of confluence (see table 1 below). The number of passages was not counted during the transfer of fragments, hence, each transfer will produce cells in passage 0. Thus, taking into account the multiple transfers of fragments, the number of cells in a low passage (up to P5 or passage 5) counted as the limit passage on their therapeutic use is practically unlimited.

**Table 1 - comparison of aspects of the invention in relation to alternative enzymatic methods of figure 1.**

| Factors for cell production | Floating non-enzymatic According to the invention | Collagenase According to the state of the art | TrypLE™ |
|---|---|---|---|
| Cell proliferation | higher | high | high |
| Tissue freezing | possible | possible | possible |
| Cell freezing | possible | possible | possible |
| Number of passages for therapy | unlimited | limited | limited |
| Number of cells | unlimited | limited | limited |
| Production on an industrial scale for biotechnological and therapeutic application | unlimited | limited | limited |

### Example 3 - Characterization by flow cytometry of cells obtained according to the process of invention, cultured in vitro.

For the analysis by flow cytometry, antibodies against cell surface molecules and their respective control isotypes were used, such as: human anti-CD45 monoclonal, (Sigma company, USA), CD90 (of BD-Pharmigen company, USA) and CD105, CD73 (of Serotec company, United Kingdom) . One million of cells are incubated with antibodies during 30 minutes on ice, washed with PBS containing 2% fetal bovine serum and 1 µM sodium azide, followed by addition of FITC (fluorescein isotiocianate) or PE (phycoerythrin). The analysis by flow cytometry is performed on a FACS (Fluorescence-Activated Cell Sorter, from Becton, Dickson and Company, USA) using CELLQuest software (from Becton, Dickson and Company, USA).

The characterization by flow cytometry in the passage discloses that the cells obtained according to the process of the invention are positive for mesenchymal stem cell markers.

### In vitro differentiation

For neuronal differentiation, inventive cells are kept confluent for a week in the 25 cm² culture bottle, containing DMEM medium supplemented with 20% Knockout serum (from Invitrogen company, USA), 100 units/mL penicillin, 100 µg/mL streptomycin, and 2 mM L-glutamine. Thus, they are collected by using a 0.05% trypsin/EDTA solution and plated at high density in 35 cm² petri dishes containing the same culture type. The neuronal differentiation is also induced by addition of all-trans retinoic acid (RA) (Sigma Company, USA). The suspension of inventive cells obtained by trypsinization is transferred to a 35 cm² petri dish, pretreated with 0.1% agarose solution (Sigma Company, USA) containing neurobasal culture medium (Invitrogen Company, USA) supplemented with B27. After 24 hours, the cells form spherical structures and neuronal differentiation is induced by addition of RA (retinoic acid) and DMSO (dimethyl sulfoxide) at a final concentration of 10⁻⁷ M and 0.05%, respectively, said medium being daily exchanged. After four days of culture under non-adherent conditions, SLS adhere on plates treated with 0.1% gelatin containing a suitable culture medium.

For adipogenic differentiation, cells were cultured in DMEM medium with 10% fetal bovine serum, 0,25 M isobutyl methyl xanthine, 10 µM insulin and 1% penicillin. The exchange of inductor medium was carried out every 3 days and maintained during 20 days. After this period, cells were fixed for 60 minutes at room temperature with 4% paraformaldehyde and washed a few times with 70% ethanol. In the following step, they were incubated at room temperature for five minutes with Oil Red O, the excess of dye was removed with a few washes with distilled water. The cells show positive staining for Von Kossa.

For chondrogenic differentiation, cells were cultured in DMEM medium with 1% bovine fetal serum, 6.25 µm insulin, 10 ng/mL TGF-µ1 and 1% penicillin. The exchange of inductor medium was carried out every 3 days and maintained during 21 days. After this period, cells were fixed with 4% paraformaldehyde at room temperature and stained with Alcian Blue. The chondrogenic differentiation can be confirmed by specific staining for safranin and toluidine.

## Claims

1. PROCESS FOR PRODUCING MULTIPOTENT STEM CELLS AND PROGENITORS, wherein the process comprises the following steps:
A - pre-preparation of one or more natural stem cell niches (NSCN) previously obtained;
B - preparation of tissue fragments of one or more NSCN;
C - promotion of stem cell (SC) and progenitor propagation by culturing floating NSCN fragments in a basal culture medium;
D - separation of SC from NSCN fragments;
E - optionally, separated NSCN fragments in D return to step C.

2. PROCESS, according to claim 1, wherein said NSCN are one or more extraembryonic tissues originated at childbirth, nervous, muscle, fat, bone, skin and organ-derived tissues, such as of liver, lungs, heart, spleen, liver, pancreas, testes, ovaries, biopsy-derived tissues and cancerous tissue having NSCN.

3. PROCESS, according to claim 1, wherein said NSCN are one or more of umbilical cord, placenta or amniotic membrane.

4. PROCESS, according to claim 1 wherein said NSCN pre-preparation in step A comprises one or more of cleaning, washing, tissue pre-cuts, grinding, compression and mild pretreatment with dissociation enzymes.

5. PROCESS, according to claim 1, wherein said fragmentation in step B is performed with integral NSCN tissue.

6. PROCESS, according to claim 1, wherein said culture medium in step C contains the nutrients necessary for cell growth contained in NSCN, particularly amino acids, proteins, 30 serum and antibiotic(s).

7. PROCESS, according to claim 1, wherein said culture medium in step C is changed as frequently as necessary to prevent pH change from acid to alkaline.

8. PROCESS, according to claim 1, wherein the fragments in step D are removed by mechanical means.

9. PROCESS, according to claim 1 wherein the stem cells and progenitors in step D are separated from tissue fragments, washed and removed after reaching a semi-confluent state, between 70% and 90%.

10. PROCESS, according to claim 1, wherein NSCN fragments, separated after said step D and returned to said step C, can be mixed with fragments obtained according to steps A and B.

## Patentansprüche

1. Verfahren zur Herstellung multipotenter Stammzellen und Progenitorzellen, wobei das Verfahren die folgenden Schritte umfasst:
A - Voraufbereitung einer oder mehrerer natürlicher Stammzellnischen (NSCN), die zuvor erhalten wurden;
B - Herstellung von Gewebefragmenten einer oder mehrerer NSCN;
C - Promotion der Stammzellen- (SC-) und Progenitorzellen-Propagation durch Kultivieren von flottierenden NSCN-Fragmente in einem basalen Kulturmedium;
D - Abtrennung von SC von NSCN-Fragmenten;
E - optional Rückführen von in D abgetrennten NSCN-Fragmenten zu Schritt C.

2. Verfahren nach Anspruch 1, wobei die NSCN ein oder mehrere extraembryonale Gewebe, die von einer Geburt stammen, von Nerven, Muskeln, Fett, Knochen, Haut und Organen stammende Gewebe, etwa von Leber, Lunge, Herz, Milz, Leber, Pankreas, Hoden, Eierstöcken, aus Biopsien stammende Gewebe oder Krebsgewebe sind, die NSCN aufweisen.

3. Verfahren nach Anspruch 1, wobei die NSCN eines oder mehrere von Nabelschnur, Plazenta oder Amnion sind.

4. Verfahren nach Anspruch 1, wobei die Voraufbereitung der NSCN in Schritt A eines oder mehrere aus Reinigen, Waschen, Gewebevorschnitten, Mahlen, Komprimieren und milde Vorbehandlung mit zersetzenden Enzymen umfasst.

5. Verfahren nach Anspruch 1, wobei die Fragmentierung in Schritt B mit integralem NSCN-Gewebe durchgeführt wird.

6. Verfahren nach Anspruch 1, wobei das Kulturmedium in Schritt C die Nährstoffe enthält, die für das Zellwachstum in den NSCN notwendig sind, insbesondere Aminosäuren, Proteine, Serum und ein oder mehrere Antibiotika.

7. Verfahren nach Anspruch 1, wobei das Kulturmedium in Schritt C so oft wie nötig ausgetauscht wird, um eine Veränderung des pH-Werts von sauer auf alkalisch zu verhindern.

8. Verfahren nach Anspruch 1, wobei die Fragmente in Schritt D durch mechanische Mittel entfernt werden.

9. Verfahren nach Anspruch 1, wobei die Stammzellen und Progenitorzellen in Schritt D von Gewebefragmenten abgetrennt, gewaschen und entfernt werden, nachdem sie einen halb fließfähigen Zustand zwischen 70 % und 90 % erreicht haben.

10. Verfahren nach Anspruch 1, wobei NSCN-Fragmente, die nach Schritt D abgetrennt und zu Schritt C zurückgeführt werden, mit Fragmenten gemischt werden können, die gemäß den Schritten A und B erhalten werden.

## Revendications

1. PROCÉDÉ DE PRODUCTION DE CELLULES SOUCHES MULTIPOTENTES ET DE PROGÉNITEURS, dans lequel le procédé comprend les étapes suivantes consistant :
A - à pré-préparer une ou plusieurs niche(s) naturelle(s) de cellules souches (NSCN) précédemment obtenues ;
B - à préparer des fragments de tissu d'une ou de plusieurs NSCN ;
C - à favoriser la propagation de cellules souches (SC) et de progéniteurs en mettant en culture des fragments flottants de NSCN dans un milieu de culture de base ;
D - à séparer des cellules SC des fragments de NSCN ;
E - éventuellement, des fragments de NSCN séparés à l'étape D retournent à l'étape C.

2. PROCÉDÉ selon la revendication 1, dans lequel lesdites NSCN sont un ou plusieurs tissu(s) extra-embryonnaire(s) trouvant son/leur origine à la naissance, des tissus nerveux, musculaires, adipeux, osseux, cutanés et issus d'organes, tels que des tissus issus du foie, des poumons, du coeur, de la rate, du foie, du pancréas, des testicules, des ovaires, d'une biopsie et un tissu cancéreux ayant des NSCN.

3. PROCÉDÉ selon la revendication 1, dans lequel lesdites NSCN sont un ou plusieurs parmi le cordon ombilical, le placenta ou la membrane amniotique.

4. PROCÉDÉ selon la revendication 1, dans lequel ladite pré-préparation de NSCN à l'étape A comprend un ou plusieurs parmi un nettoyage, un lavage, des pré-découpages de tissu, un broyage, une compression et un prétraitement doux avec des enzymes de dissociation.

5. PROCÉDÉ selon la revendication 1, dans lequel ladite fragmentation à l'étape B est réalisée avec un tissu intégral de NSCN.

6. PROCÉDÉ selon la revendication 1, dans lequel ledit milieu de culture à l'étape C contient les nutriments nécessaires à la croissance de cellules contenues dans les NSCN, en particulier des acides aminés, des protéines, du sérum et un ou plusieurs antibiotique(s).

7. PROCÉDÉ selon la revendication 1, dans lequel ledit milieu de culture à l'étape C est changé aussi souvent que nécessaire pour empêcher le changement de pH d'un pH acide à un pH alcalin.

8. PROCÉDÉ selon la revendication 1, dans lequel les fragments à l'étape D sont éliminés par des moyens mécaniques.

9. PROCÉDÉ selon la revendication 1, dans lequel les cellules souches et les progéniteurs à l'étape D sont séparés des fragments de tissu, lavés et éliminés après avoir atteint un état semi-confluent, compris entre 70% et 90%.

10. PROCÉDÉ selon la revendication 1, dans lequel des fragments de NSCN, séparés après ladite étape D et retournés à ladite étape C, peuvent être mélangés avec des fragments obtenus selon les étapes A et B.
